# EUROPEAN PATENT APPLICATION

(11) **EP 4 835 064 A1**
(43) Date of publication of application: **04.11.2026**
(21) Application number: 25305606.3
(22) Date of filing: 28.04.2025
(51) Int. Cl.: C12N 9/10, A01N 63/50

(54) **HERBICIDAL COMPOSITION AND METHOD FOR CONTROLLING UNWANTED PLANTS**

(71) Applicant: Micropep Technologies, 31320 Auzeville-Tolosane (FR)
(72) Inventor: BAROZET, Amelie, 31320 Auzeville-Tolosane (FR); BECK, Martina, 31320 Auzeville-Tolosane (FR); COURBOT, Mikael, 31320 Auzeville-Tolosane (FR); GREENHUT, Rachel, 31320 Auzeville-Tolosane (FR)
(74) Representative: Cabinet Becker et Associés

(57) **Abstract**

The invention relates to peptides that inhibits activity of 5-enolpyruvylshikimate-3-phosphate synthase (EPSPS), as well as herbicidal compositions comprising said peptides. The compositions may further comprise another EPSPS-inhibiting herbicide such as glyphosate. Methods for controlling unwanted plants, comprising applying such compositions, are also encompassed.

## Description

The present invention relates to peptides that inhibit the activity of 5-enolpyruvylshikimate-3-phosphate synthase (herein also designated "EPSP synthase" or "EPSPS"), as well as herbicidal compositions comprising such peptides.

### Technical background

Herbicides are often used to control the growth and spread of weeds or other plants that are unwanted in a particular environment. These herbicides are active at one or more target sites within a plant where they interrupt normal plant functions. Herbicides that inhibit 5-enolpyruvylshikimate-3-phosphate synthase (EPSPS), a key enzyme in the shikimate pathway used in plants for the biosynthesis of folates and aromatic amino acids, have been used for selective unwanted plants control since the 1970s.

EPSPS catalyzes the reaction of shikimate-3-phosphate (S3P) and phosphoenolpyruvate (PEP) to form 5-enolpyruvyl-shikimate-3-phosphate (EPSP). EPSP is dephosphorylated to chorismate, an essential precursor for aromatic amino acids such as phenylalanine, tyrosine and tryptophan. These amino acids are used in protein synthesis and to produce secondary metabolites such as folates, ubiquinones, and naphthoquinone.

The shikimate pathway in the chloroplast is one of the most important metabolic pathways in plants with more than 30% of the carbon fixed by photosynthesis enters the shikimate pathway (Maeda, H. and Dudareva, N. (2012) The Shikimate Pathway and Aromatic Amino Acid Biosynthesis in Plants. Annual Review of Plant Biology, 63, 73-105).

To date, glyphosate (N-phosphonomethylglycine - CAS n°1071-83-6) is the most effective EPSPS-inhibiting herbicide. The chemical structures of glyphosate and PEP are very similar. As a result, glyphosate acts as a competitive inhibitor of PEP and binds more tightly to the EPSP synthase-S3P complex than does the normal substrate PEP. However, like PEP, glyphosate has no affinity for the enzyme alone. A major difference between glyphosate and PEP is that the dissociation rate for glyphosate is 2,300 times slower than PEP. Therefore, once glyphosate binds the enzyme-substrate complex (EPSP synthase-S3P) the enzyme is essentially inactivated.

There are other factors that contribute to the herbicidal activity of glyphosate. The shikimate pathway is normally controlled by a process called feedback inhibition. In the shikimate pathway, arogenate (a product of the pathway) is a potent inhibitor of the first enzyme in the shikimate pathway, 3-deoxy-D-arabino-heptulosonate-7-phosphate synthase (DAHP synthase). Inhibition of EPSPS by glyphosate results in the decreased levels of arogenate causing the deregulation of the shikimate pathway due to increased DAHP synthase activity.

Deregulating the shikimate pathway results in the accumulation of very high levels of shikimate and S3P and, in some plant species, this accumulation can account for up to 16% of plant dry weight in sink tissues. Important building blocks for other metabolic pathways are reduced by uncontrolled carbon flow through the shikimate pathway and reduced levels of aromatic amino acids causes significant reductions in protein synthesis, leading to plant death.

Glyphosate is a systemic herbicide absorbed by plant foliage and transported throughout the plant by the phloem. Glyphosate absorption across the cuticle is moderate and, because glyphosate binds to the soil, plant uptake of glyphosate from soil is negligible.

Glyphosate is a favored unwanted plants killer due to its selective toxicity as it is considered to have low toxicity for animals because it functions by inhibiting an enzyme pathway that is specific to plants and not found in animals.

To minimize harmful plants growth, glyphosate based herbicides, are now widely used for agriculture, horticulture, viticulture, and silviculture purposes, as well as other sectors such as garden maintenance, verges, railway sidings, and land preparation for grass seeding. In both agricultural and non-agricultural environments, there are almost hundreds of commercialized glyphosate-based herbicides used in more than 100 countries, and 600 000 to 750 000 tons are used annually. Crops using glyphosate-based herbicides more frequently are soybeans, field corn, pasture, wheat, and hay (C. M. Benbrook (2016) -Trends in glyphosate herbicide use in the United States and globally- Environ. Sci. Eur., 28 (1),1-15).

The increased use of glyphosate-based herbicides since its first commercialization in 1974 for unwanted crop plants control has led to the selection of resistant biotypes. The first unwanted plant to evolve resistance to glyphosate-based herbicides was a horseweed [Conyza canadensis (L.) Cronq.] discovered in Delaware (USA) in 2000 (Dale L. Shaner and al. (2005) - A rapid in vivo shikimate accumulation assay with excised leaf discs -Weed Science, 53:769-774). To date, glyphosate resistance has been confirmed in 41 weed species worldwide, including 18 in North America (The International Survey of Herbicide Resistant Weeds, 2021). The emergence of certain herbicide-resistant unwanted plants has generated interest in developing strategies to supplement the action of primary herbicides such as glyphosate.

Thus, there is a need for novel methods to effectively control unwanted plants.

The present invention addresses these needs.

### Summary of the invention

An object of the present invention is to provide a herbicidal composition and a method for controlling unwanted plants.

More particularly, the invention provides a peptide that inhibits activity of 5-enolpyruvylshikimate-3-phosphate synthase (EPSPS), which peptide has a length of 10 to 16 amino acids and comprises a sequence SEQ ID NO: 1, SEQ ID NO: 2, or SEQ ID NO: 3 or a variant thereof defined as having at least 80% identity, preferably at least 90% identity with any of SEQ ID NO: 1 to 3, and/or 1, 2, or 3 substitution(s) of amino acids with respect to SEQ ID NO: 1 to 3.

A herbicidal composition, comprising one or more of said peptides, and optionally an agriculturally acceptable carrier, diluent or excipient, is also provided. In a preferred aspect, the composition further comprises an EPSPS-inhibiting herbicide or is intended for use in combination with another EPSPS-inhibiting herbicide, wherein the EPSPS-inhibiting herbicide is preferably glyphosate.

In a particular embodiment, the invention provides a composition comprising a combination of two to four peptides selected from the group consisting of peptides of SEQ ID NO: 1, SEQ ID NO: 4, SEQ ID NO: 7 and SEQ ID NO: 10, or variants of these peptides having an EPSPS-inhibiting activity.

In another particular embodiment, the invention provides a composition comprising a combination of two to four peptides selected from the group consisting of peptides SEQ ID NO: 2, SEQ ID NO: 5, SEQ ID NO: 8, or SEQ ID NO: 11 or variants of these peptides having an EPSPS-inhibiting activity.

In still another particular embodiment, the invention provides a composition comprising a combination of two to four peptides selected from the group consisting of peptides SEQ ID NO: 3, SEQ ID NO: 6, SEQ ID NO: 9, or SEQ ID NO: 12, or variants of these peptides having an EPSPS-inhibiting activity.

It is further provided a method of controlling one or more unwanted plants or weeds, comprising applying an effective amount of said peptide or combination of said peptides or a herbicidal composition comprising said peptide or combination of said peptides, to the unwanted plants and/or to a site of monocotyledonous or dicotyledonous weeds.

In a particular aspect, the method comprises comprising applying said peptide(s) or herbicidal composition in combination with, or sequentially with, an EPSPS-inhibiting herbicide, preferably wherein the EPSPS-inhibiting herbicide is glyphosate.

In that prospect, it is also provided a method for preparing a herbicidal composition, wherein said peptide(s) are mixed with an EPSPS-inhibiting herbicide.

### Legends to the figures

**Figure 1** is a graph that shows fresh weight raw values by condition in *A. hybridus.* A fresh weight of zero indicates a dead plant.
**Figure 2** is a graph that shows fresh weight estimates and 95% confidence by condition in *D*. *sanguinalis.*
**Figure 3** shows pictures of *A. hybridus* and *D*. *sanguinalis* at experiment end (21 days after sowing). A white cross indicates a dead plant.
**Figure 4** is graph that shows percent reduction in fresh weight (percent control) across treated conditions compared to the untreated condition across a range of weed species.
**Figure 5** is a graph that shows estimated mean percent of untreated fresh weight across all conditions, and corresponding 95% confidence interval, in *A. retroflexus.*

### Detailed description of the invention

### Definitions

As used herein, naturally occurring amino acids are identified throughout by the conventional three-letter and/or one-letter abbreviations, corresponding to the trivial name of the amino acid, in accordance with the following list: Alanine (Ala, A), Arginine (Arg, R), Asparagine (Asn, N), Aspartic acid (Asp, D), Cysteine (Cys, C), Glutamic acid (Glu, E), Glutamine (Gln, Q), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). The abbreviations are accepted in the peptide art and are recommended by the IUPAC-IUB commission in biochemical nomenclature. Naturally occurring variations of amino acids include, without limitation, gamma-glutamate (g-Glu) and isoaspartate (iso-Asp or isoD).

As used herein, the term "amino acid" further includes analogues, derivatives, and congeners of any specific amino acid referred to herein, as well as C-terminal or N-terminal protected amino acid derivatives (e.g., modified with an N-terminal, C-terminal, or side-chain protecting group, including but not limited to acetylation, formylation, methylation, amidation, esterification, PEGylation, and addition of lipids. Non-naturally occurring amino acids are well known and can be introduced into peptides of the present invention using solid phase synthesis as described below. Furthermore, the term "amino acid" includes both D- and L-amino acids. Hence, an amino acid which is identified herein by its name, three letter or one letter symbol and is not identified specifically as having the D or L configuration, is understood to assume any one of the D or L configurations.

As used herein, the term "sequence identity" or "identity" refers to the number (%) of pairs (identical amino acid residues) at positions from an alignment of two peptide sequences. Sequence identity is determined by comparing the sequences when they are aligned so as to maximize the overlap and identity while at the same time minimizing the sequence breaks. In particular, sequence identity can be determined using any of a number of global or local alignment algorithms, depending on the length of the two sequences. Sequences of similar lengths are preferably aligned using global alignment algorithms (e.g. Needleman & Wunsch, J. Mol. Biol 48:443, 1970) which optimally align the sequences along the entire length, whereas sequences of substantially different lengths are preferably aligned using a local alignment algorithm, for example the Smith-Waterman algorithm (Smith and Waterman, Adv. Appl. Math. 2:482, 1981) or the Altschul algorithm (Altschul et al. (1997) Nucleic Acids Res. 25:3389-3402; Altschul et al. (2005) FEBS J. 272:5101-5109). Alignment for the purpose of determining the percentage of amino acid sequence identity can be performed via any method known to those skilled in the art, for example using software available on websites such as BLAST (NCBI) or EMBOSS Needle (EMBL-EBI). A person skilled in the art can readily determine the appropriate parameters for measuring alignment.

The term "conservative substitution" as used herein denotes the replacement of an amino acid residue by another, without altering the overall conformation and function of the peptide, including, but not limited to, replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

As such, it should be understood that in the context of the present invention, a conservative substitution is recognized in the art as a substitution of one amino acid for another amino acid that has similar properties. Examples of amino acids having similar properties and of conservative substitutions are set out in the tables below:

**Table 1: amino acids, grouped by side chain type**

| **Amino acid groups** | **Amino acid residues** |
|---|---|
| Acidic | D and E |
| Basic | K, R and H |
| Uncharged hydrophilic | S, T, N and Q |
| Uncharged aliphatic | G, A, V, L and I |
| Uncharged nonpolar | C, M and P |
| Aromatic | F, Y and W |

**Table 2: amino acids, grouped by type of residue**

| **Amino acid groups** | **Residues** |
|---|---|
| Residues with an alcohol group | S and T |
| Aliphatic residues | I, L, V and M |
| Residues with an aromatic ring | F, H, W and Y |
| Hydrophobic residues | A, C, F, G, H, I, L, M, R, T, V, W and Y |
| Negatively charged residues | D and E |
| Positively charged residues | K, R and H |
| Polar residues | C, D, E, H, K, N, Q, R, S and T |
| Small residues | A, C, D, G, N, P, S, T and V |
| Very small residues | A, G and S |
| Flexible residues | E, Q, T, K, S, G, P, D, E and R |

**Table 3: some possible conservative amino acid substitutions**

| **Original amino acid** | **Possible conservative substitutions** |
|---|---|
| A | V, L, I |
| R | K, Q, N |
| N | Q, H, K, R |
| D | E |
| C | S |
| Q | N |
| E | D |
| H | N, Q, K, R |
| I | L, V, M, A, F |
| L | I, V, M,A, F |
| K | R, Q, N |
| M | L, F, I |
| F | L, V, I, A |
| P | G |
| S | T |
| T | S |
| W | T |
| Y | W, F, T, S |
| V | I, L, M, F, A |

### The peptides

The present invention provides peptides that inhibit the activity of 5-enolpyruvyl-shikimate-3-phosphate synthase (herein interchangeably referred to as EPSP synthase or EPSPS).

According to the invention, the peptide has a length of 10 to 16 amino acids and comprises a sequence MGEHKTRKEE (SEQ ID NO: 1), MTGKMNNCIF (SEQ ID NO: 2), or MDWTDTCKKKT (SEQ ID NO: 3) or a variant thereof defined as having at least 80% identity, preferably at least 90% identity, with any of SEQ ID NO: 1, SEQ ID NO: 2, or SEQ ID NO: 3, and/or 1, 2, or 3 substitution(s) of amino acids with respect to SEQ ID NO: 1, SEQ ID NO: 2, or SEQ ID NO: 3.

In a particular embodiment, the peptide comprises a sequence PMGEHKTRKEE (SEQ ID NO: 4), PMTGKMNNCIF (SEQ ID NO: 5), or PMDWTDTCKKKT (SEQ ID NO: 6) or a variant thereof defined as having at least 80% identity with, preferably at least 90% identity, with any of SEQ ID NO: 4, SEQ ID NO: 5, or SEQ ID NO: 6, and/or 1, and/or 1, 2, or 3 substitution(s) of amino acids with respect to SEQ ID NO: 4, SEQ ID NO: 5, or SEQ ID NO: 6.

In still another particular embodiment, the peptide comprises a sequence MGEHKTRKEED (SEQ ID NO: 7), MTGKMNNCIFD (SEQ ID NO: 8), or MDWTDTCKKKTD (SEQ ID NO: 9) or a variant thereof defined as having at least 80% identity with, preferably at least 90% identity, with any of SEQ ID NO: 7, SEQ ID NO: 9, or SEQ ID NO: 9, and/or 1, 2, or 3 substitution(s) of amino acids with respect to SEQ ID NO: 7, SEQ ID NO: 8, or SEQ ID NO: 9.

In a preferred embodiment, the peptide comprises a sequence PMGEHKTRKEED (SEQ ID NO:10), PMTGKMNNCIFD (SEQ ID NO:11), or PMDWTDTCKKKTD (SEQ ID NO: 12) or a variant thereof defined as having at least 80% identity with, preferably at least 90% identity, with any of SEQ ID NO: 10, SEQ ID NO: 11, or SEQ ID NO: 12, and/or 1, 2, or 3 substitution(s) of amino acids with respect to SEQ ID NO: 10, SEQ ID NO: 11, or SEQ ID NO: 12.

The peptides are herein described by the following name and sequence.

| **Name** | **Sequence** |
|---|---|
| MPW-01 A | MGEHKTRKEE (SEQ ID NO : 1) |
| MPW-02 A | MTGKMNNCIF (SEQ ID NO : 2) |
| MPW-03 A | MDWTDTCKKKT (SEQ ID NO : 3) |
| MPW-01 B | PMGEHKTRKEE (SEQ ID NO : 4) |
| MPW-02 B | PMTGKMNNCIF (SEQ ID NO : 5) |
| MPW-03 B | PMDWTDTCKKKT ((SEQ ID NO : 6) |
| MPW-01 C | MGEHKTRKEED (SEQ ID NO : 7) |
| MPW-02 C | MTGKMNNCIFD (SEQ ID NO : 8) |
| MPW-03 C | MDWTDTCKKKTD ((SEQ ID NO : 9) |
| MPW-01 | PMGEHKTRKEED (SEQ ID NO : 10) |
| MPW-02 | PMTGKMNNCIFD (SEQ ID NO : 11) |
| MPW-03 | PMDWTDTCKKKTD (SEQ ID NO : 12) |

The peptide preferably comprises a sequence SEQ ID NO: 1, SEQ ID NO: 2, or SEQ ID NO: 3 or a variant thereof defined as having at least 90% identity with SEQ ID NO: 1, SEQ ID NO: 2, or SEQ ID NO: 3, and/or a sequence having 1, 2, or 3 substitution(s) of amino acids with respect to SEQ ID NO: 1, SEQ ID NO: 2, or SEQ ID NO: 3, wherein the substitution(s) is/are conservative substitutions.

In a preferred embodiment the peptide is selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, and SEQ ID NO: 9.

In a still preferred embodiment, the peptide is selected from the group consisting of SEQ ID NO: 10, SEQ ID NO: 11, and SEQ ID NO: 12. The peptide of SEQ ID NO:10 is particularly preferred.

Variants of said peptides are provided, defined as having at least 80%, 85%, 90%, 95%, or 98% sequence identity with said peptides, preferably at least 90% identity with said peptides, and/or 1, 2, or 3 substitution(s) of amino acids with respect to said peptides. In a preferred embodiment, the substitutions are conservative substitutions.

The variants encompassed in the present invention retain an EPSPS-inhibiting activity.

The peptides and variants described herein advantageously inhibit the catalysis by EPSP synthase of the following reaction:

phosphoenolpyruvate (PEP) + 3-phospho shikimate (S3P) → phosphate + 5-enolpyruvylshikimate-3-phosphate (EPSP).

In a particular aspect, the peptides and variants described herein are able to prevent the closed state of EPSPS or the transition of EPSPS's conformation from open to closed state.

In a particular aspect, the peptides and variants encompassed in the present invention can bind to the interface of the EPSPS - S3P binding site, and/or to the interface of the EPSPS - glyphosate binding site .

The peptide variants preferably exhibit similar binding conformations as any of peptides SEQ ID NO: 1 to 12, binding in the interface of the two domains of EPSPS.

AlphaFold prediction metrics can be run with candidate peptide variants, and compared with the peptides comprising a sequence SEQ ID NO: 1 or 10, SEQ ID NO: 2 or 11, or SEQ ID NO: 3 or 12, respectively.

MPW-01 (SEQ ID NO: 10) was found to be, on average, at 8.08 Å of the site of fixation of S3P (S252,S253,S280,D405,S428,K342,R102,S98,K97) (Schönbrunn et al., 2001, PNAS, 98(4):1376-1380) and at 3.09 Å of the site of fixation of glyphosate (K97,N173,G175,R205,E433,R436,K503,R478) (Schönbrunn et al., 2001, supra).

MPW-02 (SEQ ID NO: 11) was found to be, on average, at 8.60 Å of the site of fixation of S3P and at 3.05 Å of the site of fixation of glyphosate.

MPW-03 (SEQ ID NO: 12) was found to be, on average, at 12.70 Å of the site of fixation of S3P and at 7.70 Å of the site of fixation of glyphosate.

In particular, the three peptides were found in the vicinity of D405,K432,R102,K97 and of K97,R205,R436,R478.

In a more particular aspect, the peptides and variants described herein, when complexed with EPSPS, are preferably located within less than 20 Å, more preferably less than 15 Å, still preferably less than 10 Å and/or at about 8 Å of the EPSPS - S3P binding site.

In a more particular aspect, the peptides and variants described herein, when complexed with EPSPS, are preferably located within less than 10 Å, more preferably less than 8 Å, still preferably less than 4 Å and/or at about 3 Å of the EPSPS - glyphosate binding site.

In another aspect of the invention, the peptides and variants described herein, when complexed with EPSPS, are preferably located in the vicinity of the glyphosate resistance site of EPSPS.

In particular, the peptides and variants described herein, when complexed with EPSPS, are preferably located within less than 20 Å, more preferably within less than 5 Å, of the threonine at position 102 of the EPSPS of *Eleusine indica* and/or the threonine at position 176 of *Amaranthus palmeri;* and/or preferably within less than 20 Å, more preferably within less than 10 Å of the proline at position 106 of the EPSPS of *Eleusine indica* and/or the proline at position 180 of *Amaranthus palmeri.*

In some aspects, the peptide comprises a tag. In some embodiments, the tag is a secretion tag, a tag that promotes entry into a plant cell, or a nuclear localization tag. In some aspects, the peptide comprises a tag at the N-terminus, the C-terminus, or both. In some aspects, the peptide comprises an amino acid sequence consisting of any of SEQ ID:1 to 12, or a variant thereof, and a tag.

In some embodiments, the peptide further comprises a cell penetrating peptide linked to the amino acid sequence of the peptide. In some embodiments, the cell penetrating peptide increases cellular delivery and activity in a cell. Cell penetrating peptides useful in the present methods are a class of short peptides with a property to translocate across cell membranes and act as nano-carriers for protein delivery into plant cells. Exemplary cell penetrating peptide families include, but are not limited to, cell penetrating peptides derived from protein transduction domains, amphipathic peptides, and synthetic cationic polypeptides, such as polylysine, polyhistidine, and polyarginine, or dendrimeric polycationic molecules. Cell penetrating peptides that may be used with the peptides of the invention include, but are not limited to, the peptide vascular endothelial-cadherin cell penetrating peptide, the transportan cell penetrating peptide, the monomer and dimer of HIV- 1 TAT basic domain cell penetrating peptide, the penetratin cell penetrating peptide, synthetic cationic homoarginine oligopeptide cell penetrating peptides (see Eudes and Chugh. (2008) Plant Signal Behav. 3:549-550), the gamma zein cell penetrating peptide (see U.S. Pat. No. 8,581,036, incorporated herein by reference in its entirety) and BP100 (Eggenberger. (2011) Chembiochem. 3;12(1):132-7). Additional non limiting examples of cell penetrating peptides, and other internalization molecules, include the Zea *mays* knotted 1 cell penetrating peptide, the Saccharomyces pombe TP 10 cell penetrating peptide, the Candida albicans Zebra cell penetrating peptide, Antennapedia sequences, TAT, Antp-3A (Antp mutant), Buforin II, MAP (model amphipathic peptide), K-FGF, Ku70, Prion, pVEC, SynBl, Pep-7, HN-1, BGSC (Bis- Guanidinium-Spermidine-Cholesterol), BGTC (Bis-Guanidinium-Tren-Cholesterol), . Additional cell penetrating peptides are described in U.S. Pat. No. 9,757,473, WO/2014/086835A1, US2017/0057997, US2020/0263189, US20200399647, and WO/2011/084061, the entireties of each of which are incorporated by reference for these peptides. In some embodiments, the cell penetrating peptide comprises a C-terminal modification, an N-terminal modification, or both. In some aspects, the peptide comprises a cell penetrating peptide linked to an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, SEQ ID NO: 9, SEQ ID NO: 10, SEQ ID NO: 11, and SEQ ID NO: 12, or a variant thereof.

In an aspect, the cell penetrating peptide is linked by a peptide bond to and located at the N-terminus of the amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, SEQ ID NO: 9, SEQ ID NO: 10 , SEQ ID NO: 11, and SEQ ID NO: 12. In another aspect, the cell penetrating peptide is located at the C-terminus of the amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, SEQ ID NO: 9, SEQ ID NO: 10, SEQ ID NO: 11, and SEQ ID NO: 12. Preferably, the cell penetrating peptide is located at the N-terminus.

In some aspects, the peptide comprises an amino acid sequence set forth in one of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, SEQ ID NO: 9, SEQ ID NO: 10 , SEQ ID NO: 11, and SEQ ID NO: 12, or a variant thereof, and an N-terminus and/or C-terminus residues. In some aspects, the peptide comprises one or more proline residues at the N-or C-terminus. In some aspects, the N-terminus and/or C-terminus residues correspond to a cleavage tag. For instance, the residue may be part of a peptide bond that is cleaved by chemical (e.g. acidic cleavage), or enzymatic reactions. In some aspects, the peptide comprises an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12, or a variant thereof, and an N-terminal proline, methionine, tryptophan, glycine or cysteine, and/or a C-terminal methionine, aspartic acid, cysteine, asparagine, or tryptophan. In a particular aspect, the peptide comprises an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, SEQ ID NO: 9, SEQ ID NO: 10 , SEQ ID NO: 11, and SEQ ID NO: 12, or a variant thereof, and an N-terminal proline and/or a C-terminal aspartic acid.

In some aspects, the peptides of the invention are produced by cleavage of a concatemer of multiple peptides that are linked together and expressed as a single polypeptide chain. In some aspects, cleavage results in a short (one to two) amino acid tag at the N and/or C terminus of the peptide. In some aspects, the two or more peptides in the concatemer are released from each other by sequence-specific chemical cleavage of the peptide bond. In some aspects, the peptide bond is an Asp-Pro bond and the sequence-specific cleavage is performed using acetic acid. In some aspects, releasing the two or more peptides from each other is performed in the absence of a chaotropic agent. In some aspects, releasing the two or more peptides from each other is performed without a column-based purification step. In some aspects, the released peptides comprise an N-terminal proline and/or a C-terminal aspartic acid. In some aspects, the released peptides comprises the amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 , SEQ ID NO: 8, SEQ ID NO: 9, SEQ ID NO: 10, SEQ ID NO: 11, and SEQ ID NO: 12, or a variant thereof, and an N-terminal proline residue and a C-terminal aspartic acid.

### Production of the peptides

The peptides described herein can be synthesized using standard synthetic methods known to those skilled in the art, for example chemical synthesis or genetic recombination.

Examples of chemical synthesis technologies are solid phase synthesis and liquid phase synthesis. As a solid phase synthesis, for example, the amino acid corresponding to the C-terminus of the peptide to be synthesized is bound to a support which is insoluble in organic solvents, and by alternate repetition of reactions, one wherein amino acids with their amino groups and side chain functional groups protected with appropriate protective groups are condensed one by one in order from the C-terminus to the N- terminus, and one where the amino acids bound to the resin or the protective group of the amino groups of the peptides are released, the peptide chain is thus extended in this manner. Solid phase synthesis methods are largely classified by the tBoc method and the Fmoc method, depending on the type of protective group used. Typically used protective groups include tBoc (t-butoxycarbonyl), CIZ (2-chlorobenzyloxycarbonyl), Br-Z (2-bromobenzyloxycarbonyl), Bzl (benzyl), Fmoc (9-fluorenylmethyloxycarbonyl), Mbh (4,4'-dimethoxybenzhydryl), Mtr (4-methoxy-2, 3, 6-trimethylbenzenesulphonyl), Trt (trityl), Tos (p- toluenesulfonyl), Z or CbZ (benzyloxycarbonyl) ,Cl2-Bzl (2,6-dichlorobenzyl); NO2 (nitro), Pmc (2,2,5,7,8-pentamethyl chromane-6-sulfonyl) and tBu (tert-butyl. After synthesis of the desired peptide, it is subjected to the de-protection reaction and cut out from the solid support. Such peptide cutting reaction may be carried with hydrogen fluoride or tri-fluoromethane sulfonic acid for the Boc method, and with TFA for the Fmoc method.

Peptide synthesis can be carried out on automated solid phase peptide synthesis instruments including, without limitation, the Applied Biosystems 431 A, 433 A synthesizers and Peptide Technologies Symphony or large scale Sonata or CEM Liberty automated solid phase peptide synthesizers. The use of alternative peptide synthesis instruments is also contemplated.

Alternatively, the peptide may be synthesized using recombinant techniques. The peptides of the present invention may be also prepared by using recombinant expression systems followed by separation and purification of the recombinantly prepared peptides. Generally, this involves inserting an encoding nucleic acid molecule into an expression system to which the molecule is heterologous (i.e., not normally present). One or more desired nucleic acid molecules encoding a peptide of the invention may be inserted into the vector. The heterologous nucleic acid molecule is inserted into the expression system or vector in proper sense (5'-3') orientation and correct reading frame relative to the promoter and any other 5' and 3' regulatory molecules.

Thus, in an aspect, the invention relates to a nucleic acid and/or a genetic construct comprising or consisting of a nucleic acid sequence encoding a peptide according to the invention, polynucleotides with nucleic acid sequences complementary to one of the above sequences and sequences hybridizing to said polynucleotides under stringent conditions.

Expression (including transcription and translation) of a peptide by the DNA construct may be regulated with respect to the level of expression. A number of heterologous regulatory sequences (e.g., promoters and enhancers) are available for controlling the expression of the DNA construct. Once a suitable expression vector is selected, the desired nucleic acid sequences are cloned into the vector using standard cloning procedures in the art. The vector is then introduced to a suitable host.

In an aspect, a concatemeric expression strategy is used to produce the peptide(s) according to the invention. In other words, a coding nucleic acid including several copies of a nucleotide sequence encoding one of the peptides of the invention are integrated into a suitable expression vector. The peptides are then cleaved, preferably by sequence-specific cleavage.

The invention further relates to a genetic construct consisting of or comprising a polynucleotide as defined herein, and regulatory sequences (such as a suitable promoter(s), enhancer(s), terminator(s), etc.) allowing the expression (e.g. transcription and translation) of a peptide according to the invention in a host cell.

Thus, in another aspect, the invention relates to a host or host cell that expresses (or that under suitable circumstances is capable of expressing) a peptide of the invention; and/or that contains a polynucleotide or genetic construct as described herein.

A variety of host-vector systems may be utilized to recombinantly express the peptides of the present invention. Primarily, the vector system must be compatible with the host used. Host-vector systems include, without limitation, the following: bacteria transformed with bacteriophage DNA, plasmid DNA, or cosmid DNA; microorganisms such as yeast containing yeast vectors; mammalian cell systems infected with virus (e.g., vaccinia virus, adenovirus, etc.); insect cell systems infected with virus (e.g., baculovirus); and plant cells infected *by Agrobacterium.* The expression elements of these vectors vary in their strength and specificities. Depending upon the host-vector system utilized, any one of a number of suitable transcription and translation elements can be used to carry out this and other aspects of the present invention.

In a preferred aspect, the host cell that expresses the peptide of the invention is an *E. coli* cell, optionally a host cell as described in the international application WO 2024/175788.

The method of producing the peptide recombinantly may optionally comprise the steps of purifying said peptide.

Purified peptides may be obtained by several methods. The peptide is preferably produced in purified form (preferably at least about 80% or 85% pure, more preferably at least about 90% or 95% pure) by conventional techniques. Depending on whether the recombinant host cell is made to secrete the peptide into growth medium, the peptide can be isolated and purified by centrifugation (to separate cellular components from supernatant containing the secreted peptide) followed by sequential ammonium sulfate precipitation of the supernatant. The fraction containing the peptide is subjected to gel filtration in an appropriately sized dextran or polyacrylamide column to separate the peptides from other proteins. If necessary, the peptide fraction may be further purified by HPLC. Alternatively, if the peptide of interest is not secreted, it can be isolated from the recombinant cells using standard isolation and purification schemes. This includes disrupting the cells (e.g., by sonication, freezing, French press, etc.) and then recovering the peptide from the cellular debris. Purification can be achieved using standard centrifugation, precipitation, and purification procedures. The use of purification tags can simplify this process.

### Herbicidal compositions

The peptide(s) described herein are then advantageously formulated into herbicidal compositions.

It is thus also provided a herbicidal composition comprising a peptide as defined herein, alone or in combination with other peptides, and optionally an agriculturally acceptable carrier, excipient or diluent.

As used herein, the term "agriculturally acceptable" in reference to a carrier, material, ingredient or substance of a composition comprising a peptide as defined herein means that the carrier, material, ingredient or substance, as the case may be, (i) is compatible with other ingredients of the composition at least for the purpose in which the composition will be used, (ii) can be included in the composition to effectively and viably deliver the peptide as defined herein to a plant, plant part, plant seed, or plant growth medium (e.g., soil), and (iii) is not deleterious to a plant, plant part, or plant seed to which the composition will be associated or applied (at least in the manner and amount in which it will be applied to, or associated with, the plant, plant part, or plant seed). An agriculturally acceptable carrier may include a soil-compatible carrier, a seed-compatible carrier, and/or a foliar-compatible carrier. As used herein, the term "soil-compatible carrier" refers to a material that can be added or applied to a soil without causing/having an unduly adverse effect on plant yield, soil structure, soil drainage, or the like. The term "seed-compatible carrier" refers to a material that can be added or applied to a seed without causing/having an unduly adverse effect on the seed, seed germination, the plant that grows from the seed, or the like. The term "foliar-compatible carrier" refers to a material that can be added or applied to an above ground portion of a plant or plant part without causing/having an unduly adverse effect on plant yield, plant health, or the like. Selection of appropriate carrier materials will depend on the intended application(s). The carrier material(s) may be selected and/or combined to provide a composition or formulation in the form of a liquid, gel, slurry, or solid. Compositions may comprise one or more liquid and/or gel carriers, and/or one or more aqueous and/or non-aqueous solvents.

The peptides or compositions as herein described may be combined with known active compounds which are based on the inhibition of EPSPS.

In a particular aspect, the herbicidal composition comprises a peptide as defined herein and an EPSPS-inhibiting agent or EPSPS-inhibiting herbicide. In a particular aspect, the EPSPS-inhibiting agent is glyphosate (N-phosphonomethylglycine - CAS n°1071-83-6) or an agriculturally acceptable salt thereof, such as glyphosate-ammonium, -isopropylammonium, - diammonium, -dimethylammonium, -potassium, -sodium and -trimesium.

In an aspect, the herbicidal composition comprising a peptide as defined herein, and optionally comprising an EPSPS-inhibiting herbicide, in particular glyphosate, comprises at least one further active compound from the group of herbicides.

Herbicides include substances that affect plant growth, development and/or reproductive ability and substances used, in particular, to neutralize or destroy a plant species that is harmful or troublesome during the production or storage of agricultural products.

In an aspect, the composition further comprises herbicidal mixing partners. Examples of such herbicidal mixing partners include, without being limited to: glutamine synthetase inhibitors, p-hydroxyphenylpyruvate dioxygenase inhibitors, phytoene desaturase inhibitors, photosystem I inhibitors, photosystem II inhibitors, protoporphyrinogen oxidase inhibitors, acetochlor, acifluorfen, acifluorfen-sodium, aclonifen, alachlor, allidochlor, alloxydim, alloxydim-sodium, ametryn, amicarbazone, amidochlor, amidosulfuron, 4-amino-3-chloro-5-fluoro-6-(7-fluoro-1H-indol-6-yl)pyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bixlozone, bromacil, bromobutide, bromofenoxim, bromoxynil, bromoxynil-butyrate, - potassium, -heptanoate and -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, 1-{2-chloro-3-[(3-cyclopropyl-5-hydroxy-1-methyl-1H-pyrazol-4-yl)carbonyl]-6-(trifluoromethyl)phenyl}piperidin-2-one, 4-{2-chloro-3-[(3,5-dimethyl-1H-pyrazol-1-yl)methyl]-4-(methylsulfonyl)benzoyl}-1,3-dimethyl-1H-pyrazol-5-yl 1,3-dimethyl-1H-pyrazole-4-carboxylate, chlorfenac, chlorfenac-sodium, chlorfenprop, chlorflurenol, chlorflurenol-methyl, chloridazon, chlorimuron, chlorimuron-ethyl, 2-[2-chloro-4-(methylsulfonyl)-3-(morpholin-4-ylmethyl)benzoyl]-3-hydroxycyclohex-2-en-1-one, 4-{2-chloro-4-(methylsulfonyl)-3-[(2,2,2-trifluorethoxy)methyl]benzoyl}-1-ethyl-1H-pyrazol-5-yl 1,3-dimethyl-1H-pyrazole-4-carboxylate, chlorophthalim, chlorotoluron, chlorthal-dimethyl, chlorsulfuron, 3-[5-chloro-4-(trifluoromethyl)pyridin-2-yl]-4-hydroxy-1-methylimidazolidin-2-one, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium and -trolamine, 2,4-DB, 2,4-DB-butyl, - dimethylammonium, isooctyl, -potassium and -sodium, daimuron (dymron), dalapon, dazomet, n-decanol, desmedipham, detosyl-pyrazolate (DTP), dicamba, dichlobenil, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, 3-(2,6-dimethylphenyl)-6-[(2-hydroxy-6-oxocyclohex-1-en-1-yl)carbonyl]-1-methylquinazoline-2,4(1H,3H)-dione, 1,3-dimethyl-4-[2-(methylsulfonyl)-4-(trifluoromethyl)benzoyl]-1H-pyrazol-5-yl 1,3-dimethyl-1H-pyrazole-4-carboxylate, dimetrasulfuron, dinitramine, dinoterb, diphenamid, diquat, diquat-dibromide, dithiopyr, diuron, DMPA, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, ethyl [(3-{2-chloro-4-fluoro-5-[3-methyl-2,6-dioxo-4-(trifluoromethyl)-3,6-dihydropyrimidin-1(2H)-yl]phenoxy}pyridin-2-yl)oxy]acetate, F-9960, F-5231, i.e. N-[2-chloro-4-fluor-5-[4-(3-fluoropropyl)-4,5-dihydro-5-oxo-1H-tetrazol-1-yl]phenyl]ethanesulfonamide, F-7967, i.e. 3-[7-chloro-5-fluor-2-(trifluoromethyl)-1H-benzimidazol-4-yl]-1-methyl-6-(trifluoromethyl)pyrimidine-2,4(1H,3H)-dione, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, florpyrauxifen, florpyrauxifen-benzyl, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium and - methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium,H-9201i.e.O-(2,4-dimethyl-6-nitrophenyl)-O-ethyl isopropylphosphoramidothioate, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(dimethoxyphosphoryl)ethyl (2,4-dichlorophenoxy)acetate, 4-hydroxy-1-methoxy-5-methyl-3-[4-(trifluoromethyl)pyridin-2-yl]imidazolidin-2-one, 4-hydroxy-1-methyl-3-[4-(trifluoromethyl)pyridin-2-yl]imidazolidin-2-one, (5-hydroxy-1-methyl-1H-pyrazol-4-yl)(3,3,4-trimethyl-1,1-dioxido-2,3-dihydro-1-benzothiophen-5-yl)methanone, 6-[(2-hydroxy-6-oxocyclohex-1-en-1-yl)carbonyl]-1,5-dimethyl-3-(2-methylphenyl)quinazoline-2,4(1H,3H)-dione, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxynil-octanoate, - potassium and sodium, ipfencarbazone, isoproturon, isouron, isoxaben, isoxaflutole, karbutilate, KUH-043, i.e. 3-({[5-(difluoromethyl)-1-methyl-3-(trifluoromethyl)-1H-pyrazol-4-yl]methyl}sulfonyl)-5,5-dimethyl-4,5-dihydro-1,2-oxazole, ketospiradox, lactofen, lenacil, linuron, MCPA, MCPA-butotyl, -dimethylammonium, -2-ethylhexyl, -isopropylammonium, - potassium and -sodium, MCPB, MCPB-methyl, -ethyl and -sodium, mecoprop, mecoprop-sodium, and -butotyl, mecoprop-P, mecoprop-P-butotyl, -dimethylammonium, -2-ethylhexyl and -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, 2-({2-[(2-methoxyethoxy)methyl]-6-(trifluoromethyl)pyridin-3-yl}carbonyl)cyclohexane-1,3-dione, methyl isothiocyanate, 1-methyl-4-[(3,3,4-trimethyl-1,1-dioxido-2,3-dihydro-1-benzothiophen-5-yl)carbonyl]-1H-pyrazol-5-yl propane-1-sulfonate, metobromuron, metolachlor, S-metolachlor, metosulam, metoxuron, metribuzin, metsulfuron, metsulfuron-methyl, molinat, monolinuron, monosulfuron, monosulfuron ester, MT-5950, i.e. N-[3-chloro-4-(1-methylethyl)-phenyl]-2-methylpentanamide, NGGC-011, napropamide, NC-310, i.e. 4-(2,4-dichlorobenzoyl)-1-methyl-5-benzyloxypyrazole, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefon, oxotrione (lancotrione), oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, QYM-201, QYR-301, rimsulfuron, saflufenacil, sethoxydim, siduron, simazine, simetryn, sulcotrion, sulfentrazone, sulfometuron, sulfometuron-methyl, sulfosulfuron, SYN-523, SYP-249, i.e. 1-ethoxy-3-methyl-1-oxobut-3-en-2-yl 5-[2-chloro-4-(trifluoromethyl)phenoxy]-2-nitrobenzoate, SYP-300, i.e. 1-[7-fluoro-3-oxo-4-(prop-2-yn-1-yl)-3,4-dihydro-2H-1,4-benzoxazin-6-yl]-3-propyl-2-thioxoimidazolidine-4,5-dione, 2,3,6-TBA, TCA (trifluoroacetic acid), TCA-sodium, tebuthiuron, tefuryltrione, tembotrione, tepraloxydim, terbacil, terbucarb, terbumeton, terbuthylazin, terbutryn, tetflupyrolimet, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, and ZJ-0862, i.e. 3,4-dichloro-N-{2-[(4,6-dimethoxypyrimidin-2-yl)oxy]benzyl}aniline.

In an aspect, other mixing partners may be included in the herbicidal composition of the invention, including other active compounds such as one or more insecticide(s), one or more acaricide(s), and/or one or more fungicide(s).

In an aspect, the herbicidal composition of the invention may also comprise one or more active safener(s), one or more fertilizer(s) and/or one or more plant growth regulator(s).

In a particular aspect, the herbicidal composition of the invention comprises a plant growth regulator. Plant growth regulators include substances with positive or negative effects on plant growth and development. They include, but are not limited to: phytotoxins, substances that promote plant growth and/or increase yield, such as phytohormones and/or fertilizers, and substances that enhance biotic and abiotic stress tolerance of plants, such as safeners that protect plants against herbicides or compounds that modulate drought tolerance.

Examples of plant growth regulators as possible mixing partners include, without being limited to: acibenzolar, acibenzolar-S-methyl, 5-aminolevulinic acid, ancymidol, 6-benzylaminopurine, brassinolide, catechol, chlormequat chloride, cloprop, cyclanilide, 3-(cycloprop-1-enyl)propionic acid, daminozide, dazomet, n-decanol, dikegulac, dikegulac-sodium, endothal, endothal-dipotassium, -disodium, and mono(N,N-dimethylalkylammonium), ethephon, flumetralin, flurenol, flurenol-butyl, flurprimidol, forchlorfenuron, gibberellic acid, inabenfide, indole-3-acetic acid (IAA), 4-indol-3-ylbutyric acid, isoprothiolane, probenazole, jasmonic acid, jasmonic acid methyl ester, maleic hydrazide, mepiquat chloride, 1-methylcyclopropene, 2-(1-naphthyl)acetamide, 1-naphthylacetic acid, 2-naphthyloxyacetic acid, nitrophenolate mixture, 4-oxo-4[(2-phenylethyl)amino]butyric acid, paclobutrazole, N-phenylphthalamic acid, prohexadione, prohexadione-calcium, prohydrojasmone, salicylic acid, strigolactone, tecnazene, thidiazuron, triacontanol, trinexapac, trinexapac-ethyl, tsitodef, uniconazole, uniconazole-P.

In an aspect, the herbicidal composition of the invention comprises an active safener. Active safeners are substances or preparations, which are added to plant protection products such as herbicides, fungicides, insecticides, acaricides, nematicides, growth regulators, repellents, or rodenticides, to eliminate or reduce phytotoxic effects of the plant protection product on certain plants.

Examples of active safeners as possible mixing partners include, without being limited to: compounds which eliminate the unwanted herbicidal action of herbicides on crop plants such as for example 3-phenylisoxazoline-5-carboxamides compounds described in EP0520371 and US12185723, mefenpyr-diethyl, cyprosulfamide, isoxadifen-ethyl, cloquintocet-mexyl, benoxacor, dichlormid and metcamifen.

The herbicidal composition of the invention can be formulated in various ways, according to the biological and/or physicochemical parameters required. Possible formulations include, for example, wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), dispersions based on oil or water, oil-miscible solutions, capsule suspensions (CS), dusting products (DP), dressings, granules for scattering and soil application, granules (GR) in the form of microgranules, spray granules, absorption and adsorption granules, water-dispersible granules (WG), water-soluble granules (SG), ULV formulations, microcapsules and waxes.

In an aspect, the herbicidal composition of the invention comprises one or more of the following agriculturally acceptable carrier, diluent or excipients: customary stickers, wetters, dispersants, emulsifiers, penetrants, preservatives, antifoams, antifreeze agents and solvents, fillers, carriers and dyes, defoamers, pH adjusters, adjuvant spreaders, humectants, compatibilizers, evaporation inhibitors, paraffinic oils, vegetable oils, buffering agents, hydrotrope agents, rheology modifiers, UV protectants, antioxidants, chelating agents and/or agents which influence the pH and the viscosity.

The herbicidal composition of the invention may contain 0.1 to 99% by weight, especially 0.1 to 95% by weight, of the peptide(s) described herein. In wettable powders, the peptide concentration is, for example, about 10 to 90% by weight, the remainder to 100% by weight consisting of agriculturally acceptable carrier, diluent or excipients as well as mixing partners as described herein. In emulsifiable concentrates, the peptide concentration may be about 1% to 90% and preferably 5% to 80% by weight. Formulations in the form of dusts comprise 1% to 30% by weight of peptide, preferably usually 5% to 20% by weight of peptide; sprayable solutions contain about 0.05% to 80% by weight, preferably 2% to 50% by weight of peptide. In the case of water-dispersible granules, the peptide content depends partially on whether the peptide is in liquid or solid form and on which granulation auxiliaries, fillers, etc., are used. In the water-dispersible granules, the content of peptide is, for example, between 1 and 95% by weight, preferably between 10 and 80% by weight.

The herbicidal composition of the invention may be provided in the form of a finished formulation (ready-to-use) or as a tank mixture.

Thus, the invention also relates to a method for preparing a herbicidal composition, wherein the peptide as defined herein is mixed with an EPSPS-inhibiting herbicide. In a particular aspect, the EPSPS-inhibiting herbicide is glyphosate.

In another aspect, a herbicidal composition of the invention, for example comprising a peptide of the invention and an EPSPS-inhibiting herbicide, is provided for tank mixing with another mixing partner as defined herein. Thus, the invention also relates to a method for preparing a tank mixture, wherein a herbicidal composition of the invention comprising a peptide of the invention and an EPSPS-inhibiting herbicide is mixed with other mixing partners.

In a particular embodiment, the herbicidal composition may be a seed-dressing formulation or a seed-coating formulation, comprising a peptide as described herein.

The herbicidal composition may then similarly contain an agriculturally acceptable carrier, diluent or excipients as well as mixing partners, formulated as solutions, emulsions, suspensions, powders, foams, slurries or other coating compositions for seed, and also ultra-low volume (ULV) formulations. These formulations are produced in a known manner, by mixing the peptide(s) with customary additives, for example customary extenders and solvents or diluents, dyes, wetting agents, dispersants, emulsifiers, antifoams, preservatives, secondary thickeners, adhesives, gibberellins, and also water.

Dyes which may be present in the seed-dressing formulations usable in accordance with the invention are all dyes which are customary for such purposes. It is possible to use either pigments, which are sparingly soluble in water, or dyes, which are soluble in water.

Useful wetting agents which may be present in the seed-dressing or seed-coating formulations usable in accordance with the invention are all substances which promote wetting and which are customary for the formulation of agrochemically active compounds. Alkyl naphthalenesulfonates, such as diisopropyl or diisobutyl naphthalenesulfonates, can be used with preference.

Suitable dispersants and/or emulsifiers which may be present in the seed-dressing or seed-coating formulations usable in accordance with the invention are all nonionic, anionic and cationic dispersants customary for the formulation of agrochemically active compounds. Preference is given to using nonionic or anionic dispersants or mixtures of nonionic or anionic dispersants. Suitable nonionic dispersants include especially ethylene oxide/propylene oxide block polymers, alkylphenol polyglycol ethers and tristyrylphenol polyglycol ethers, and the phosphated or sulfated derivatives thereof. Suitable anionic dispersants are especially lignosulfonates, polyacrylic acid salts and arylsulfonate-formaldehyde condensates. Antifoams which may be present in the seed-dressing or seed-coating formulations usable in accordance with the invention are all foam-inhibiting substances customary for the formulation of agrochemically active compounds. Silicone antifoams and magnesium stearate can be used with preference.

Preservatives which may be present in the seed-dressing or seed-coating formulations usable in accordance with the invention are all substances usable for such purposes in agrochemical compositions. Examples include dichlorophene and benzyl alcohol hemiformal.

Secondary thickeners which may be present in the seed-dressing or seed-coating formulations usable in accordance with the invention are all substances usable for such purposes in agrochemical compositions. Preferred examples include cellulose derivatives, acrylic acid derivatives, xanthan, modified clays and finely divided silica.

Useful stickers which may be present in the seed-dressing or seed-coating formulations usable in accordance with the invention are all customary binders usable in seed-dressing products. Preferred examples include polyvinylpyrrolidone, polyvinyl acetate, polyvinyl alcohol and tylose.

In a preferred aspect, the herbicidal composition of the invention is tolerated by the useful plants or crops.

### Methods for controlling unwanted plants

The present invention also relates to a product comprising a peptide as defined herein or to a herbicidal composition as defined herein, for controlling one or more monocotyledonous or dicotyledonous weeds. By "control" or "controlling" is meant killing, eliminating, reducing in numbers, reducing the effects, preventing weeds from being established and/or preventing or reducing growth of unwanted plants or weeds.

The present invention provides a method for controlling unwanted plants or weeds, comprising applying an effective amount of a peptide as described herein, or a herbicidal composition comprising said peptide, to the unwanted plants and/or to a site of monocotyledonous or dicotyledonous weeds.

Generally speaking, a plant, plant part or plant seed may be transgenic or non-transgenic and/or contain one or more genetic changes or mutations. A "plant" refers to a plant at any stage of development including an embryo, seedling, and mature plant whether grown or developed from a seed, regenerated from a cultured tissue, or propagated in any manner. As used herein, a "plant part" refers to any organ or intact tissue of a plant, such as a meristem, shoot organ/structure (e.g., leaf, stem or node), root, flower or floral organ/structure (e.g., bract, sepal, petal, stamen, carpel, anther and ovule), seed (e.g., embryo, endosperm, and seed coat), fruit (e.g., the mature ovary), propagule, or other plant tissues (e.g., vascular tissue, dermal tissue, ground tissue, and the like), or any portion thereof.

The term "weed" refers to plants that compete with cultivated plants, plants of economic interest or plants that are useful for humans as, e.g., food, cooking spices, medicines, construction materials, or ornamentation.

The term "unwanted plant" refers to a plant occurring in unwanted environments, or a plant the sprouting or growth of which is not desired, and which it is desired to destroy and of which it is desired to limit the development.

Unwanted plants are, for example, harmful plants such as monocotyledonous or dicotyledonous weeds or unwanted crop plants and their seed (for example grains, seeds or vegetative propagules such as tubers or shoot parts with buds).

In a particular aspect, the growth, development, or reproductive ability of the unwanted plant treated by the method is reduced, or said plant is more sensitive to an EPSPS inhibitor herbicide when compared to an untreated plant.

The peptides or composition as defined herein can be utilized and applied, for example, prior to sowing (if appropriate also by incorporation into the soil), prior to emergence or after emergence of the plant. Specific examples of some representatives of the monocotyledonous and dicotyledonous weed flora which can be controlled by the peptide(s) or herbicidal composition(s) of the invention are as follows, though the enumeration is not intended to impose a restriction to particular species.

Unwanted plants can include monocotyledonous harmful plants of the genera: *Aegilops, Agropyron, Agrostis, Alopecurus, Apera, Avena, Brachiaria, Bromus, Cenchrus, Commelina, Cynodon, Cyperus, Oactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.*

Unwanted plants can also include dicotyledonous weeds of the genera: *Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.*

In a particular aspect, the unwanted plants to be treated by the method of the invention belong to a genus selected from the group consisting of *Digitaria, Amaranthus, Ipomoea, Kochia* and/or *Cyperus.*

The peptides or herbicidal composition of the invention can be used to control harmful plants in crop and ornamental plants. Preference is given to using the herbicidal composition in economically important crops of useful and ornamental plants such as for example maize, soybean, cotton, *Brassica* oil seeds such as *Brassica napus* (e.g. Canola), *Brassica rapa, B. juncea* (e.g. (field) mustard) and *Brassica carinata,* rice, wheat, sugar beet, sugar cane, oats, rye, barley, millet and sorghum, triticale, flax, grapes and various fruit and vegetables from various botanic taxa, for example Rosaceae sp. (for example pome fruits such as apples and pears, but also stone fruits such as apricots, cherries, almonds and peaches, and berry fruits such as strawberries), *Ribesioidae* sp., *Juglandaceae* sp., *Betulaceae* sp., *Anacardiaceae* sp., *Fagaceae* sp., *Moraceae* sp., *Oleaceae* sp., *Actinidaceae* sp., *Lauraceae* sp., *Musaceae* sp. (for example banana trees and plantations), *Rubiaceae* sp. (for example coffee), *Theaceae* sp., *Sterculiceae* sp., *Rutaceae* sp. (for example lemons, oranges and grapefruit); *Solanaceae* sp. (for example tomatoes, potatoes, peppers, aubergines), *Liliaceae* sp., *Compositae* sp. (for example lettuce, artichokes and chicory-including root chicory, endive or common chicory), *Umbelliferae* sp. (for example carrots, parsley, celery and celeriac), Cucurbitaceae sp. (for example cucumbers-including gherkins, pumpkins, watermelons, calabashes and melons), *Alliaceae* sp. (for example leeks and onions), *Cruciferae* sp. (for example white cabbage, red cabbage, broccoli, cauliflower, Brussels sprouts, pak choi, kohlrabi, radishes, horseradish, cress and Chinese cabbage), *Leguminosae* sp. (for example peanuts, peas, and beans-for example runner beans and broad beans), *Chenopodiaceae* sp. (for example Swiss chard, fodder beet, spinach, beetroot), *Malvaceae* (for example okra), *Asparagaceae* (for example asparagus); useful plants and ornamental plants in the garden and woods; and genetically modified varieties of each of these plants.

In a particular aspect, the peptides or herbicidal composition of the invention can be used in crops of rice, corn, sunflower, wheat, cotton, or soybean.

In a particular aspect, wild plant species and plant cultivars, or those obtained by conventional biological breeding techniques, such as crossing or protoplast fusion, and parts thereof, are treated by the peptides or herbicidal composition of the invention. In a further particular aspect, transgenic plants and plant cultivars obtained by genetic engineering methods, if appropriate in combination with conventional methods (genetically modified organisms), and parts thereof are treated.

The peptides or herbicidal composition of the invention can be used in crops of genetically modified organisms (GMOs), e.g. plants or seeds. Genetically modified plants (or transgenic plants) are plants in which a heterologous gene has been stably integrated into the genome. The term "heterologous gene" means essentially a gene which is provided or assembled outside the plant and which, upon introduction into the nuclear genome, the chloroplast genome or the mitochondrial genome, imparts to the transformed plant novel or improved agronomical or other traits because it expresses a protein or polypeptide of interest or another gene which is present in the plant, or other genes which are present in the plant are downregulated or switched off (for example by means of antisense technology, co-suppression technology or RNA interference technology [RNAi]). A heterologous gene that is located in the genome is also called a transgene. A transgene that is defined by its specific presence in the plant genome is called a transformation or transgenic event.

In general, the transgenic plants are characterized by particular advantageous properties, for example by resistances to certain active compounds used in the agrochemical industry, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material. Further particular properties lie in tolerance or resistance to abiotic stress factors, for example heat, cold, drought, salinity and ultraviolet radiation.

Conventional ways of producing novel plants which have modified properties in comparison to existing plants consist, for example, in traditional cultivation methods and the generation of mutants. Alternatively, novel plants with altered properties can be generated with the aid of recombinant methods. What has been described are, for example, several cases of genetic modifications of crop plants for the purpose of modifying the starch synthesized in the plants, transgenic crop plants which are resistant to certain herbicides of the glufosinate type, of the glyphosate type or of the sulfonylurea type Transgenic plants which are resistant to combinations or mixtures of these herbicides through "gene stacking" have also been described, such as transgenic crop plants, for example maize or soybean with the trade name or the designation Optimum^{™} GAT^{™} (Glyphosate ALS Tolerant), transgenic crop plants, for example cotton, capable of producing *Bacillus thuringiensis* toxins (Bt toxins), which make the plants resistant to particular pests (EP0142924, EP0193259), transgenic crop plants having a modified fatty acid composition, genetically modified crop plants having novel constituents or secondary metabolites, for example novel phytoalexins, which cause an increase in disease resistance (EP0309862, EP0464461), genetically modified plants having reduced photorespiration, which have higher yields and higher stress tolerance (EP0305398), transgenic crop plants which produce pharmaceutically or diagnostically important proteins ("molecular pharming"), transgenic crop plants which feature higher yields or better quality, or transgenic crop plants which are distinguished by a combination, for example, of the abovementioned novel properties ("gene stacking").

The herbicidal composition of the invention can be used in transgenic crops which are resistant to herbicides which inhibit essential plant enzymes, for example acetolactate synthases (ALS), EPSP synthases, glutamine synthases (GS) or hydroxyphenylpyruvate dioxygenases (HPPD), or to growth regulators, for example 2,4-D, dicamba or to herbicides from the group of the sulfonylureas, the glyphosates, glufosinates or benzoylisoxazoles and analogous active compounds, or to any desired combinations of these active compounds. The herbicidal composition of the invention can be used with particular preference in transgenic crop plants which are resistant to a combination of glyphosates and glufosinates, glyphosates and sulfonylureas or imidazolinones.

The herbicidal composition of the invention can be used more preferably with plants or plant cultivars (obtained by plant biotechnology methods such as genetic engineering) which are herbicide-tolerant plants, i.e. plants made tolerant to one or more given herbicides. Such plants can be obtained either by genetic transformation, or by selection of plants containing a mutation imparting such herbicide tolerance.

Herbicide-tolerant plants are for example glyphosate-tolerant plants, i.e. plants made tolerant to the herbicide glyphosate or salts thereof. Plants can be made tolerant to glyphosate by various methods. Thus, for example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium* (Comai et al., 1983, Science, 221, 370-371), the CP4 gene of the bacterium *Agrobacterium sp.* (Barry et al., 1992, Curr. Topics Plant Physiol. 7, 139-145), the genes encoding a petunia EPSPS (Shah et al., 1986, Science 233, 478-481), a tomato EPSPS (Gasser et al., 1988, J. Biol. Chem. 263, 4280-4289) or an Eleusine EPSPS (WO 01/66704). It can also be a mutated EPSPS.

Glyphosate-tolerant plants can also be obtained by expressing a gene that encodes a glyphosate oxidoreductase enzyme. Glyphosate-tolerant plants can also be obtained by expressing a gene that encodes a glyphosate acetyltransferase enzyme. Glyphosate-tolerant plants can also be obtained by selecting plants containing naturally-occurring mutations of the abovementioned genes. Plants which express EPSPS genes which impart glyphosate tolerance have been described. Plants which express other genes which impart glyphosate tolerance, for example decarboxylase genes, have been described.

Particularly useful transgenic plants which can be treated in accordance with the invention are for examples of herbicide-tolerant plants which may be mentioned include maize varieties, cotton varieties and soybean varieties which are available under the following trade names: Roundup Ready^{®} (tolerance to glyphosates, for example maize, cotton, soybeans), Optimum^{™} GAT^{™} (tolerance to glyphosate ALS for example maize, soybean), Liberty Link^{®} (tolerance to phosphinothricin, for example oilseed rape) and Clearfield^{®} (tolerance to imidazolinone for example rice, sunflower, wheat, maize).

In an aspect, the herbicidal composition of the invention may be used against glyphosate herbicide resistant unwanted plants or weeds. In a particular aspect, it is used against EPSPS-resistant unwanted plants or weeds.

In an aspect, the herbicidal composition of the invention is used as herbicides for controlling harmful plants in seed plants and in seed transgenic plants.

In that case, treatment of the seed protects not only the seed itself but also the resulting plants after emergence from harmful plants. In this way, the immediate treatment of the crop at the time of sowing or shortly thereafter can be dispensed with.

The herbicidal composition of the invention, which may be a seed-dressing or seed-coating formulation, is suitable for protection of seed of any plant variety which is used in agriculture, in the greenhouse, in forests or in horticulture and viticulture. In particular, the herbicidal composition, which may be a seed-dressing or seed-coating formulation, is suitable for protection of cereal seeds (such as wheat, barley, rye, triticale, sorghum/millet and oats), maize, cotton, soybeans, rice, potatoes, sunflower, bean, coffee, beet (for example sugar beet and fodder beet), peanut, oilseed rape, poppy, olive, coconut, cocoa, sugar cane, tobacco, vegetables (such as tomato, cucumbers, onions and lettuce), turf and ornamentals. In a more particular aspect, cereal seeds (such as wheat, barley, rye, triticale and oats), maize seeds and rice seeds are treated. In a particular aspect, seeds of rice, corn, sunflower, wheat, cotton or soybean are treated by the peptide or herbicidal composition of the invention.

In a particular aspect, the peptides or herbicidal composition of the invention is used to treat transgenic seeds.

In particular, the herbicidal composition is used for the treatment of the seeds of transgenic crops/plants which are resistant to herbicides which inhibit essential plant enzymes, for example acetolactate synthases (ALS), EPSP synthases, glutamine synthases (GS) or hydroxyphenylpyruvate dioxygenases (HPPD), or to growth regulators, for example 2,4-D, dicamba or to herbicides from the group of the sulfonylureas, the glyphosates, glufosinates or benzoylisoxazoles and analogous active compounds, or to any desired combinations of these active compounds. The herbicidal composition of the invention can be used with particular preference to treat the seeds of transgenic crop plants which are resistant to a combination of glyphosates and glufosinates, glyphosates and sulfonylureas or imidazolinones. In a particular aspect, the seeds of transgenic rice, transgenic corn, transgenic sunflower, transgenic wheat, transgenic cotton or transgenic soybean are treated by the peptides or by the herbicidal composition which may be a seed-dressing formulation, as described herein.

In a particular aspect, the herbicidal composition, which may be a seed-dressing formulation, as described herein, is applied to the seed alone or in a suitable formulation. In general, the seed can be treated at any time between harvest and sowing. In an aspect, the seeds to be treated have been separated from the plant and freed from cobs, shells, stalks, coats, hairs or the flesh of the fruits. In a more particular aspect, the seeds to be treated have been harvested, cleaned and dried down to a moisture content of less than 15% by weight. Alternatively, it is also possible to treat seeds which, after drying, for example, have been treated with water and then dried again.

In a particular aspect, the amount of the herbicidal composition of the invention and/or further additives applied to the seed is chosen such that the germination of the seed is not impaired and the plant which arises therefrom is not damaged.

In a particular aspect, the herbicidal composition of the invention is applied directly, i.e. without containing any other components and without having been diluted. In a preferred aspect, the herbicidal composition of the invention is applied to the seed in the form of a suitable formulation. Suitable formulations and methods for seed treatment are known to those skilled in the art and are described, for example, in the following documents: U.S. Pat. No. 4,272,417, US 4,245,432, US 4,808,430; US5,876,739, US 2003/0176428, WO 2002/080675, WO 2002/028186.

The herbicidal composition usable in accordance with the invention can be used, either directly or after previously having been diluted with water, for the treatment of a wide range of different seeds, including the seeds of transgenic plants.

For the treatment of seed with the herbicidal composition usable in accordance with the invention or with the preparations prepared therefrom by addition of water, useful equipment is all mixing units usable customarily for seed dressing. Specifically, the seed dressing or seed coating procedure is to place the seed into a mixer, to add the particular desired amount of seed-dressing formulations, either as such or after prior dilution with water, and to mix them until the formulation is distributed homogeneously on the seed. If appropriate, this is followed by a drying operation.

The herbicidal composition of the invention given good plant compatibility, favorable homeotherm toxicity and good environmental compatibility, is suitable for protection of plants and plant organs, for increasing harvest yields, and for improving the quality of the harvested crop. They can preferably be used as crop protection agents. They are active against normally sensitive and resistant species and also against all or specific stages of development.

In an aspect, treatment of the plants and plant parts with the herbicidal composition of the invention is carried out directly or by action on their surroundings, habitat or storage space using customary treatment methods, for example by dipping, spraying, atomizing, irrigating, evaporating, dusting, fogging, broadcasting, foaming, painting, spreading-on, watering (drenching), drip irrigating and, in the case of propagation material, in particular in the case of seeds, furthermore by dry seed treatment, wet seed treatment, slurry seed treatment, incrusting, coating with one or more coats, etc. In a further aspect, the peptides are applied by the ultra-low volume method or by injecting the peptide into the soil.

The herbicidal composition of the invention can be applied in the form of wettable powders, emulsifiable concentrates, sprayable solutions, dusting products or granules in the customary formulations.

In a further aspect the present disclosure relates to a tank mix composition suitable to be sprayed using standard agricultural spray equipment, wherein said tank mix composition is obtainable by mixing a herbicidal composition according to the present disclosure with an appropriate amount of water, optionally adding one or more further ingredients selected from the group of further herbicidal active ingredients and further auxiliaries.

Preferably, the total amount of water for obtaining such a tank mix composition according to the present disclosure is in the range of about 50 liters to about 1,000 liters, more preferably of about 100 liters to about 400 liters, per kg of herbicidal composition according to the present disclosure.

For application, the formulations in commercial form are, if appropriate, diluted in a customary manner, for example in the case of wettable powders, emulsifiable concentrates, dispersions and water-dispersible granules with water, dust-type preparations, granules for soil application or granules for scattering and sprayable solutions are not normally diluted further with other inert substances prior to application.

The required application rate of the herbicidal composition of the invention varies according to the external conditions such as, inter alia, temperature, humidity and the type of herbicide used. It can vary within wide limits, for example between 0.001 and 10.0 kg/ha or more of active substance, but it is preferably between 0.005 and 5 kg/ha, more preferably in the range of from 0.01 to 1.5 kg/ha, particularly preferably in the range of from 0.05 to 1 kg/ha g/ha. This applies both to the pre-emergence and the post-emergence application.

The person skilled in the art, for example a weed control practitioner can readily select and determine the application rates of herbicidal composition according to the present disclosure that are herbicidally effective on particular species at particular growth stages in particular environmental conditions.

### Minicells

The present invention also encompasses a minicell comprising a peptide as described herein, or a nucleic acid as described herein, encoding said peptide.

The term "minicell" encompasses derivatives of eubacterial cells that lack a chromosome; derivatives of archaebacterial cells that lack their chromosome(s), and anucleate derivatives of eukaryotic cells that lack a nucleus and consequently a chromosome.

Examples of minicells are described in international application WO2019060903.

The peptides described herein may be internally expressed in the minicell or encapsulated within the minicell. In an aspect, they are displayed at the surface of the minicells. In an embodiment, the peptide described herein is encapsulated within the minicell and is released over time, preferably in a controlled manner.

The minicell can be formulated in a herbicidal composition. The present invention thus discloses a herbicidal composition comprising said minicell. The herbicidal composition comprising a minicell may comprise an EPSPS-inhibiting herbicide, in particular glyphosate. It may also comprise another herbicide as listed above, as well as other mixing partners, including other active compounds such as one or more insecticide(s), one or more acaricide(s), and/or one or more fungicide(s).

In an aspect, the herbicidal composition comprising a minicell of the invention may also comprise one or more active safener(s), one or more fertilizer(s) and/or one or more plant growth regulator(s).

The herbicidal composition comprising a minicell of the invention can be formulated in various ways, according to the biological and/or physicochemical parameters required. Possible formulations include, for example, wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), dispersions based on oil or water, oil-miscible solutions, capsule suspensions (CS), dusting products (DP), dressings, granules for scattering and soil application, granules (GR) in the form of microgranules, spray granules, absorption and adsorption granules, water-dispersible granules (WG), water-soluble granules (SG), ULV formulations, microcapsules and waxes. The herbicidal composition comprising a minicell of the invention can also be in the form of a customary seed-dressing or seed-coating formulation.

The present invention also discloses a method for delivering one or more peptides of the invention to a weed or unwanted plant, comprising applying a minicell as described herein. Also encompassed is a method of delivering one or more peptides of the invention to a weed or unwanted plant comprising applying a herbicidal composition, wherein said herbicidal composition comprises a minicell as described herein.

The figures and examples illustrate the invention without limiting its scope.

### EXAMPLES

### Materials and methods

### Materials

Seeds of weedy populations of *Digitaria sanguinalis, Amaranthus hybridus, Amaranthus retroflexus,* mixed *Ipomoea spp.* (*I. lacunosa* and *I. hederacea*), and *Kochia scoparia* as well as tubers of *Cyperus esculentus* were sourced from Azlin Seeds (Leland, MS). Synthetic peptides MPW-01 (SEQ ID NO: 10), MPW-02 (SEQ ID NO: 11), MPW-03 (SEQ ID NO: 12) were produced by Smartbox Biotechnology (St Egrave, France). All chemicals used were commercially sourced.

### Plant Propagation

Plants were grown in Sun Gro - Sunshine #5 Propagation mix (60-80% peat moss, perlite, limestone). *D. sanguinalis* seeds were germinated in standard greenhouse flats and transplanted when plants reached V1 growth stage at 7 days after sowing (DAS) into 2.5 inch diameter pots with one plant per pot. All other species were sown directly into 2.5 inch pots and thinned after emergence and again just prior to the start of the experiment to achieve one plant per pot. Seeding rates varied by species and 30% extra pots were sown or transplanted with the aim of obtaining optimal uniformity in plant growth stage and to remove any seed contaminants. Sowing dates were aligned to reach V2 for monocots and 2 true leaves for dicots on the same day with the exception of *K. scoparia* which had 5 true leaves at the experiment start (T0). At T0, 30% of the pots were discarded based on uniformity of plant growth stage. The remaining pots were randomly assigned to and grouped by condition. Each condition contained 9 (*Ipomoea spp*.) or 12 (all other spp.) plants per species grouped into sets of 3 or 4 plants per experimental unit (EU).

### Growth Conditions

Experiments were conducted in a greenhouse with day/night temperature set to 30/28°C (+/-3°C), relative humidity limit of 55% and daylength of 16h with supplemental metal halide and high pressure sodium lighting. Automated ebb and flow flood irrigation was used with no supplemental fertilizer. Irrigation occurred 4 times per week throughout the course of the experiment with additional hand watering during germination and emergence to maintain a moist soil surface.

### Treatment Preparation

Treatments were prepared fresh just prior to application according to the schedule and doses in Table 4. The glyphosate herbicide product used was Roundup Powermax^{®} 3 (Bayer Crop Science) and doses aimed for sublethal control which varied by species (i.e., 70% biomass control with low lethality). Glyphosate treatments were prepared by diluting Roundup Powermax^{®} 3 in DI water. Synthetic peptides was solubilized in ultrapure water in cryotubes at a concentration of 5 g/L. Glyphosate and peptide concentrations and doses across experiments, species, and conditions are listed in Table 5. Peptide and blank treatments for all species were prepared with TM-024 which consists of a pH buffer (MES 1M pH 7.0), an antifoam agent (Silcolapse 411), and adjuvants to enhance spreading, humectancy, and penetration (Atlox AL-2575LF and Tween 20), and DI water (concentrations listed in Table 6).

**Table 4. Condition details including treatments, schedule, and doses for three experiments (Exp.).**

| **Exp.** | **Condition** | **T0** | **T0 + 1 Day** | **T0 + 3 days** |
|---|---|---|---|---|
| 1 | Untreated | | | |
| | Glyphosate* | Glyphosate | | |
| | Glyphosate+Blank | Glyphosate | Blank-TM-024 | Blank-TM-024 |
| | Glyphosate+MPW-01 | Glyphosate | MPW-01-TM-024 100g ai/ha | MPW-01-TM-024 100g ai/ha |
| 2 | Untreated | | | |
| | Glyphosate+Blank | Glyphosate | Blank-TM-024 | Blank-TM-024 |
| | Glyphosate+MPW-01 | Glyphosate | MPW-01-TM-024 40g ai/ha | MPW-01-TM-024 40g ai/ha |
| | Glyphosate+MPW-02 | Glyphosate | MPW-02-TM-024 40g ai/ha | MPW-02-TM-024 40g ai/ha |
| 3 | Untreated | | | |
| | Glyphosate Alone | Glyphosate | | |
| | Glyphosate+Blank | Glyphosate | Blank-TM-024 | Blank-TM-024 |
| | Glyphosate+MPW-01 | Glyphosate | MPW-01-TM-024 40g ai/ha | MPW-01-TM-024 40g ai/ha |
| | Glyphosate+MPW-02 | Glyphosate | MPW-02-TM-024 40g ai/ha | MPW-02-TM-024 40g ai/ha |
| | Glyphosate+MPW-03 | Glyphosate | MPW-03-TM-024 40g ai/ha | MPW-03-TM-024 40g ai/ha |

| | | | | |
|---|---|---|---|---|
| *Glyphosate product used was Roundup Powermax ^{®} 3 and dose varied by species. | | | | |

**Table 5: Herbicide and peptide preparation concentrations (g a.i./L) and doses (g a.i./ha) applied across different species in three experiments. Doses listed are for a single treatment application.**

| | | **Glyphosate** | | **Peptide** | |
|---|---|---|---|---|---|
| Exp. | **Species** | **Dose** | **Concentration** | **Dose** | **Concentration** |
| 1 | *A. hybridus* | 57 | 0.314 | 100 | 0.552 |
| | *D. sanguinalis* | 250 | 1.379 | 100 | 0.552 |
| 2 | *A. hybridus* | 57 | 0.314 | 40 | 0.221 |
| | *K. scoparia* | 420 | 2.317 | 40 | 0.221 |
| | *Ipomoea spp.* | 420 | 2.317 | 40 | 0.221 |
| | *C. esculentus* | 1133 | 6.251 | 40 | 0.221 |
| 3 | *A. retroflexus* | 57 | 0.314 | 40 | 0.221 |

**Table 6 : Blank-TM-024 components and concentrations.**

| **Component** | **Concentration (g/L)** |
|---|---|
| Atlox AL-2575LF | 2 |
| Tween 20 | 0.5 |
| MES 1M pH 7.0 Buffer | 10 |
| Silcolapse 411 | 0.1 |
| DMSO | 2 |
| DI Water | QS |

### Treatment Application

Glyphosate treatments were applied at T0. Peptide and blank treatments were applied in two consecutive treatments on 1 and 3 days after T0. Prior to treatment application, the greenhouse air handler was shut down to reduce air movement during application for roughly 30 minutes. For all treatments, hand-held perfume sprayers were used to apply treatments at a volume roughly equivalent to 180 L/ha by spraying 2 pumps per plant at a height of 6 inches above the canopy.

### Example 1

MPW-01 (SEQ ID NO: 10) was tested on *A. hybridus* and *D*. *sanguinalis* according to the conditions listed in Table 4 and Table 5.

### Data collection

Baseline measurements were collected at T0 just prior to treatment application. Overhead photos were collected in a photo booth for baseline leaf area measurement of dicot species. Leaf area was collected from photos using Fiji by 1) setting the scale to the known pot diameter; 2) using the Huang thresholding method adjust the hue, saturation, and brightness to identify only green colored pixels of leaves and stems; and 3) selecting and measuring leaf area of individual plants using the wand and measuring tools. Plant heights were collected by measuring from the soil surface to the tip of the longest leaf blade when straightened in *D. sanguinalis.*

Individual plant fresh weights were collected for all species at the experiment end (11 days after T0). Plants were scored as dead or alive based on visual inspection of the leaves, stem, and meristem tissue.

### Statistical analysis

An analysis of variance (ANOVA) was attempted for both species, fitting the terms Condition and Block to compare fresh weights across conditions. The fresh weight of each plant was square-root transformed prior to analysis. The mean transformed fresh weight was calculated for each experimental unit and the analysis was conducted at the experimental unit level. No covariate was fitted (the relationship between final Fresh Weight and Baseline measurement was weak). Due to the existence of dead plants in Glyphosate + Blank treated conditions, the assumptions of ANOVA are violated and therefore the analysis focused mainly on comparing conditions based on the count of dead vs alive plants at the experiment end. A Fisher's exact test to compare the proportion of dead plants for each Glyphosate condition to Glyphosate + Blank was conducted for each species. No adjustment for multiple comparisons were applied.

### Results

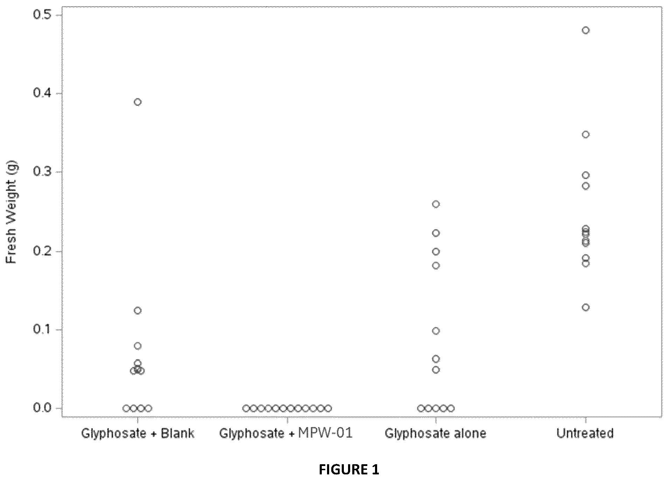

The existence of many dead plants (zero fresh weight) in all glyphosate conditions in *A. hybridus* severely violates the assumptions of ANOVA. Therefore, ANOVA was not conducted for this species. Raw fresh weights by condition in **Figure 1** show that generally, untreated plants were the largest followed by those treated with glyphosate alone and glyphosate + blank whereas plants treated with glyphosate + MPW-01 had the lowest fresh weight where all plants died (fresh weights of zero). Percent mortality (percentage of dead plants) was compared between each glyphosate condition to Glyphosate + Blank using a 2-sided Fisher's exact test (Table 7). Glyphosate + MPW-01 resulted in 100% plant mortality vs 33.3% in Glyphosate + Blank (p = 0.0013). Glyphosate + Blank vs Glyphosate alone did not significantly differ (p = 1.0).

**Table 7: Percent mortality across conditions and species, and p-values from 2-sided Fisher's exact test.**

| **Species** | **Treatment** | **Number Dead n(%)** | **p-value vs. Glyphosate + Blank** |
|---|---|---|---|
| *A. hybridus* | Glyphosate + Blank | 4 (33.3%) | |
| | Glyphosate + MPW-01 | 12 (100.0%) | 0.0013 |
| | Glyphosate alone | 5 (41.7%) | 1 |
| | Untreated | 0 (0.0%) | |
| *D. sanguinalis* | Glyphosate + Blank | 0 (0.0%) | |
| | Glyphosate + MPW-01 | 6 (50.0%) | 0.0037 |
| | Glyphosate alone | 1 (8.3%) | 1 |
| | Untreated | 0 (0.0%) | |

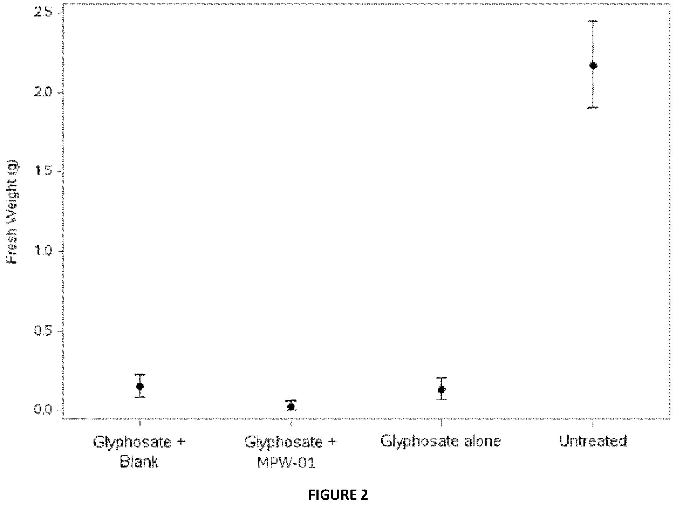

The existence of some dead plants for most conditions for D. *sanguinalis* results in a violation of the assumptions of ANOVA. Nonetheless, this violation was considered mild and an ANOVA of the square root transformed fresh weight was carried out. Results of ANOVA showed an effect of Condition (p < 0.0001) but not for Block (p = 0.4714). There is evidence of a reduction in final fresh weight for Glyphosate + MPW-01 when compared to Glyphosate + Blank, where MPW-01 resulted in -83.8% difference in fresh weight vs blank when applied following glyphosate (p < 0.0001) **(Table 8,** **Figure 2****).** Fisher's exact test showed that Glyphosate + MPW-01 resulted in 50% plant mortality vs 0% in Glyphosate + Blank (p = 0.0137) (Table 7). Glyphosate + Blank vs Glyphosate alone did not significantly differ (p = 1.0).

**Table 8. ANOVA results, estimates, and percent differences with 95% confidence levels (CL) of square-root transformed fresh weight in D. sanguinalis.**

| **Treatment** | **Transformed Mean** | **Detransformed Mean** | | | **Percent Difference vs Glyphosate + Blank** | | |
|---|---|---|---|---|---|---|---|
| | | **Estimate** | **Lower95% CL** | **Upper95% CL** | **Estimate** | **Lower95% CL** | **Upper95% CL** |
| Glyphosate + Blank Glyphosate + | 0.384 | 0.147 | 0.085 | 0.226 | | | |
| MPW-01 | 0.154 | 0.024 | 0.004 | 0.061 | -83.8 | -99.6 | -45.2 |
| Glyphosate alone | 0.359 | 0.129 | 0.072 | 0.204 | -12.3 | -64.3 | 62.5 |
| Untreated | 1.472 | 2.166 | 1.904 | 2.444 | | | |
| F-Test probability (Treatment) | <0.0001 | | | | | | |
| F-Test probability (Block) | 0.4714 | | | | | | |
| Variance | 0.005 | | | | | | |
| SD | 0.071 | | | | | | |
| 5% LSD | 0.130 | | | | | | |

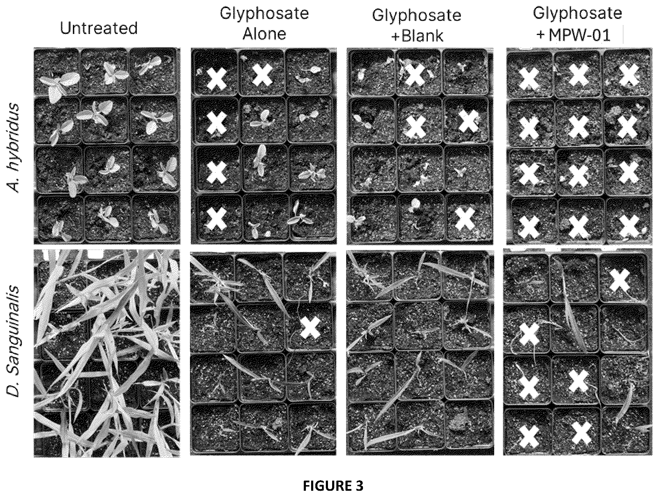

In summary, there is evidence that Glyphosate + MPW-01 results in more dead plants and lower biomass compared to Glyphosate + Blank or Glyphosate alone in both species. This experiment also demonstrates that complete control (100% mortality) can be achieved in co-application with a sublethal dose of glyphosate (+67% vs Glyphosate + Blank) in some species. Across both species, these results were visually striking **(****Figure 3****).**

### Example 2

MPW-01 (SEQ ID NO: 10) and MPW-03 (SEQ ID NO: 12) were tested on *Amaranthus hybridus, Cyperus esculentus, Ipomoea spp.* and *Kochia scoparia* according to the conditions listed in Table 4 and Table 5.

### Data collection

Individual plant fresh weights were collected for all species at the experiment end (15 days after T0). Plants were scored as dead or alive based on visual inspection of the leaves, stem, and meristem tissue.

### Statistical analysis

Fresh weight means were calculated for each condition and species. Level of control was calculated as the percent difference in fresh weight mean for each condition compared that of Untreated. The difference in control levels between Glyphosate + Blank and Glyphosate + Peptide were calculated as a percent.

### Results

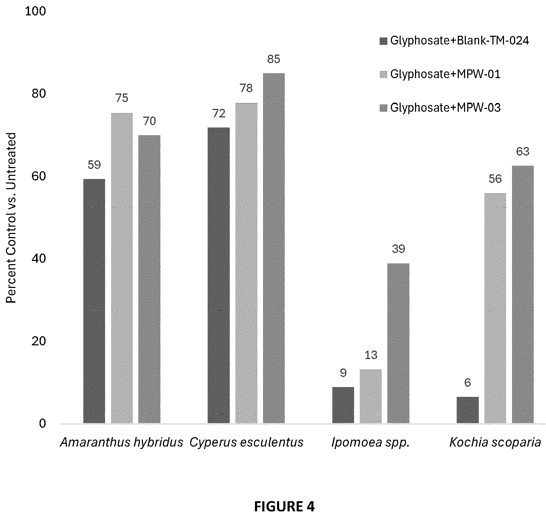

Across the 4 species tested, the mean percent reduction in fresh weight (percent control) compared to fresh weight of Untreated ranged from 13 to 78% with Glyphosate + MPW-01 and from 39 to 85% with Glyphosate + MPW-03 **(Table 9,** **Figure 4****).** The highest percent control of 85% was reached in C. *esculentus* with Glyphosate + MPW-03. The combined mean percent control compared to Untreated across all species was 36.6, 55.6, and 64.1 for Glyphosate + blank, Glyphosate + MPW-01, and Glyphosate + MPW-03, respectively. The difference in percent control compared to Glyphosate + Blank ranged from 4 to 56% control across species. The mean combined difference in percent control vs Glyphosate + Blank (i.e., the added control provided by the peptide) was 19.0 and 27.5% for MPW-01 and MPW-03, respectively.

In summary, there is evidence that both MPW-01 and MPW-03 can provide added control when applied following a sublethal dose of glyphosate across a wide range of weed species. MPW-03 was the most effective, especially in *Ipomoea spp* and *K. scoparia.*

**Table 9: Fresh weight means and percent control vs Untreated and vs Blank by condition and species.**

| **Species** | **Condition** | **Fresh Weight (g)** | **Percent Control vs Untreated** | **Diff.** in **Control vs Gly+Blank** |
|---|---|---|---|---|
| *Amaranthus hybridus* | Untreated | 0.23 | 0.0 | - |
| | Glyphosate+Blank-TM-024 | 0.09 | 59.3 | 0.0 |
| | Glyphosate+MPW-01 | 0.06 | 75.5 | 16.1 |
| | Glyphosate+MPW-03 | 0.07 | 70.0 | 10.6 |
| *Cyperus esculentus* | Untreated | 1.09 | 0.0 | - |
| | Glyphosate+Blank-TM-024 | 0.31 | 71.8 | 0.0 |
| | Glyphosate+MPW-01 | 0.24 | 77.8 | 6.0 |
| | Glyphosate+MPW-03 | 0.16 | 85.0 | 13.1 |
| *Ipomoea spp.* | Untreated | 1.33 | 0.0 | - |
| | Glyphosate+Blank-TM-024 | 1.21 | 8.8 | 0.0 |
| | Glyphosate+MPW-01 | 1.16 | 13.1 | 4.3 |
| | Glyphosate+MPW-03 | 0.81 | 38.9 | 30.1 |
| *Kochia scoparia* | Untreated | 0.39 | 0.0 | - |
| | Glyphosate+Blank-TM-024 | 0.36 | 6.4 | 0.0 |
| | Glyphosate+MPW-01 | 0.17 | 55.9 | 49.5 |
| | Glyphosate+MPW-03 | 0.15 | 62.5 | 56.1 |
| Combined | Untreated | 0.76 | 0.00 | - |
| | Glyphosate+Blank-TM-024 | 0.49 | 36.61 | 0.00 |
| | Glyphosate+MPW-01 | 0.41 | 55.59 | 18.97 |
| | Glyphosate+MPW-03 | 0.30 | 64.08 | 27.47 |

### Example 3

MPW-01 (SEQ ID NO: 10), MPW-02 (SEQ ID NO: 11) and MPW-03 (SEQ ID NO: 12) were tested on the amaranth species *Amaranthus retroflexus* according to the conditions listed in Table 4 and Table 5.

### Data collection

Baseline measurements were collected at T0 just prior to treatment application. End point leaf area measurements were also collected. Individual plant fresh weights were collected at the experiment end (10 days after T0). Plants were scored as dead or alive based on visual inspection of the leaves, stem, and meristem tissue.

### Statistical analysis

An ANOVA was conducted fitting the terms Condition and Block to compare fresh weights across conditions. The fresh weight of each plant was square-root transformed prior to analysis and the mean transformed fresh weight was calculated for each experimental unit. Analyses were conducted at the experimental unit level. No baseline covariates were fitted since there was no evidence of a relationship with baseline leaf area. The high proportion of dead plants for some conditions present a violation of the assumptions of ANOVA (as the data in truncated at zero). This is improved by an analysis on the square-root transformed scale. No adjustment was made for multiple comparisons.

### Results

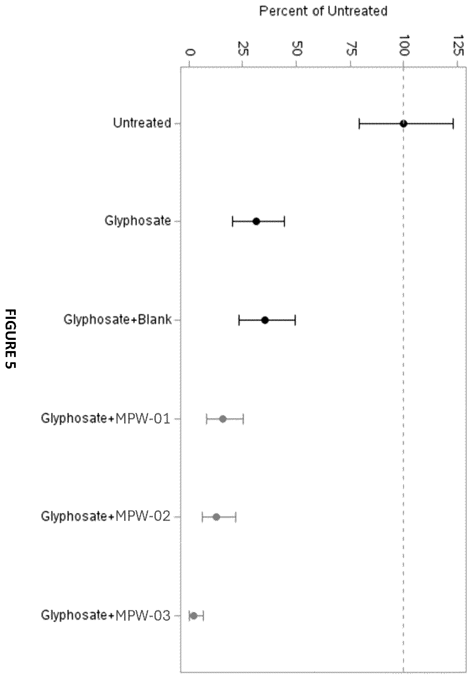

Results of ANOVA showed an effect of Condition (p < 0.0001) but not for Block (p = 0.10). The percent of untreated fresh weight mean (where Untreated is 100%) for Glyphosate Alone and Glyphosate + Blank was 31 and 35%, respectively. In comparison, for each Glyphosate + peptide condition the percent of untreated fresh weight mean was 16, 13, and 2% for MPW-01, MPW-02, and MPW-03, respectively **(****Figure 5****).** Simply put, Glyphosate + Peptide conditions were 84 to 98% lower in fresh weight compared to Untreated. The percent difference vs Glyphosate + Blank for each Glyphosate + peptide condition were -55, -63, and -94% for MPW-01, MPW-02, and MPW-03, respectively (Table 10). Essentially, Glyphosate + Peptide conditions were 55 to 94% lower in fresh weight compared to Glyphosate + Blank.

**Table 10. ANOVA results, estimates, and percent differences with 95% confidence levels (CL) of square-root transformed fresh weight in A. retroflexus.**

| **Treatment** | **Transformed Mean** | **Detransformed Mean** | | | **Percent Difference vs Glyphosate + Blank** | | |
|---|---|---|---|---|---|---|---|
| | | **Estimate** | **Lower95% CL** | **Upper 95% CL** | **Estimate** | **Lower95% CL** | **Upper 95% CL** |
| Untreated | 0.69 | 0.481 | 0.382 | 0.592 | 184 | 103 | 279 |
| Glyphosate | 0.39 | 0.150 | 0.097 | 0.214 | -11 | -54 | 44 |
| Glyphosate+Blank | 0.41 | 0.169 | 0.113 | 0.237 | | | |
| Glyphosate+MPW-01 | 0.27 | 0.075 | 0.040 | 0.123 | -55 | -83 | -14 |
| Glyphosate+MPW-02 | 0.25 | 0.062 | 0.030 | 0.106 | -63 | -88 | -25 |
| Glyphosate+MPW-03 | 0.10 | 0.010 | 0.001 | 0.032 | -94 | -100 | -74 |
| F-Test probability (Treatment) | <0.0001 | | | | | | |
| F-Test probability (Block) | 0.50 | | | | | | |
| Variance | 0.0035 | | | | | | |
| SD | 0.059 | | | | | | |
| 5% LSD | 0.10 | | | | | | |

In summary, there is evidence that Glyphosate in co-application with MPW-01, MPW-02, and MPW-03 resulted in lower fresh weight than Glyphosate + Blank. There is no convincing evidence of a difference between Glyphosate alone and Glyphosate + Blank, suggesting that the blank does not provide any added control without peptide.

## Claims

1. A peptide that inhibits activity of 5-enolpyruvylshikimate-3-phosphate synthase (EPSPS), which peptide has a length of 10 to 16 amino acids and comprises a sequence MGEHKTRKEE (SEQ ID NO: 1), MTGKMNNCIF (SEQ ID NO: 2), or MDWTDTCKKKT (SEQ ID NO: 3) or a variant thereof defined as having at least 80% identity with, and/or 1, 2, or 3 substitution(s) of amino acids with respect to SEQ ID NO: 1, 2 or 3.

2. The peptide of claim 1, which peptide comprises any of SEQ ID NO: 4 to 12, or a variant thereof defined as having at least 80%, preferably at least 90%, identity with any of SEQ ID NO: 4 to 12 and/or 1, 2, or 3 substitution(s) of amino acids with respect to any of SEQ ID NO: 4 to 12.

3. The peptide of claim 2, comprising a sequence having 1, 2, or 3 substitution(s) of amino acids with respect to any of SEQ ID NO: 4 to 12, wherein the substitution(s) is/are conservative substitutions.

4. The peptide of claim 1, consisting of any of SEQ ID NO: 1 to 12.

5. The peptide of claim 4, consisting of SEQ ID NO: 10.

6. A herbicidal composition, comprising one or more peptides of any of claims 1 to 5, and optionally an agriculturally acceptable carrier, diluent or excipient.

7. The herbicidal composition of claim 6, which composition comprises at least one peptide that consists of SEQ ID NO: 1, 4, 7, or 10, or a combination of two to four of said peptides.

8. The herbicidal composition according to claim 6 or 7, further comprising an EPSPS-inhibiting herbicide.

9. The herbicidal composition according to claim 8, wherein the EPSPS-inhibiting herbicide is glyphosate.

10. A herbicidal composition according to any of claims 6 to 9, further comprising at least one further active compound from the group of insecticides, acaricides, herbicides, fungicides, safeners and/or growth regulators.

11. A method of controlling one or more unwanted plants or weeds, comprising applying an effective amount of a peptide according to claim 1 to 5 or a herbicidal composition according to claim 6 to 10 to unwanted plants and/or to a site of monocotyledonous or dicotyledonous weeds.

12. The method of claim 11, wherein the peptide or herbicidal composition is applied to control unwanted plants or weeds in a rice, corn, sunflower, wheat, cotton or soybean culture.

13. The method of claim 11 or 12, wherein the unwanted plants belong to a genus selected from the group consisting of *Digitaria, Amaranthus, Ipomoea, Kochia* and *Cyperus.*

14. The method of any of claims 11 to 13, comprising applying a peptide according to claim 1 to 5 or a herbicidal composition according to claim 6 to 10, in combination with, or sequentially with, an EPSPS-inhibiting herbicide, preferably wherein the EPSPS-inhibiting herbicide is glyphosate.

15. A method for preparing a herbicidal composition, wherein the peptide of any of claims 1-5 is mixed with an EPSPS-inhibiting herbicide.
